(19) Europäisches Patentamt
European Patent Office
Office européen des brevets

(11) EP 1 413 301 A1

(12) DEMANDE DE BREVET EUROPEEN

(43) Date de publication:
**28.04.2004 Bulletin 2004/18**

(51) Int Cl.[7]: **A61K 31/155**

(21) Numéro de dépôt: **02356210.1**

(22) Date de dépôt: **24.10.2002**

(84) Etats contractants désignés:
**AT BE BG CH CY CZ DE DK EE ES FI FR GB GR IE IT LI LU MC NL PT SE SK TR**
Etats d'extension désignés:
**AL LT LV MK RO SI**

(71) Demandeur: **Bayer CropScience SA**
**69266 Lyon Cedex 09 (FR)**

(72) Inventeurs:
- **Vors, Jean-Pierre**
  **69009 Lyon (FR)**
- **O'Neill, Elizabeth**
  **69300 Caluire (FR)**
- **Labourdette, Gilbert**
  **71600 Paray le Monial (FR)**
- **Mansfield, Gilian**
  **69004 Lyon (FR)**
- **Pillmoor, John**
  **York YO19 5PF (GB)**
- **Barchietto, Thierry**
  **95380 Puiseux-en-France (FR)**

(74) Mandataire: **Mérigeault, Shona et al**
**Bayer CropScience SA**
**Département Propriété Industrielle**
**B.P. 9163**
**69263 Lyon Cedex 09 (FR)**

(54) **Médicaments antifongiques à base de dérivés d'arylamidine**

(57) La présente invention a pour objet de nouveaux médicaments antifongiques à base de dérivés de $N_2$-phénylamidine et éventuellement d'au moins un agent synergique anti-fongique.

EP 1 413 301 A1

## Description

La présente invention a pour objet de nouveaux médicaments antifongiques.

Plus précisément, la présente invention a pour objet de nouveaux médicaments antifongiques à base de dérivés de $N_2$-phénylamidine et éventuellement d'au moins un agent synergique anti-fongique.

Par médicament antifongique, on entend une composition pharmaceutique destinée à être administrée à un humain ou un animal.

La demande internationale WO-00/46184 décrit un ou plusieurs dérivés de $N_2$-phénylamidine. De tels composés sont utilisés dans le domaine agricole comme composés fongicides.

La société demanderesse a mis en évidence de façon tout à fait inattendue que les dérivés de $N_2$-phénylamidine constituaient également des composés antifongiques de choix, tant chez l'être humain que chez l'animal.

Aussi, l'un des objectifs essentiels de la présente invention est de fournir un nouveau médicament antifongique à base de dérivés de $N_2$-phénylamidine.

Un autre objectif essentiel de l'invention est de fournir un nouveau médicament antifongique tout à fait performant, notamment en ce qui concerne son efficacité contre les champignons.

Un autre objectif essentiel de l'invention est de fournir un nouveau médicament fongicide combinant de façon synergique au moins un dérivés de $N_2$-phénylamidine et au moins un autre composé connu comme ayant une activité antifongique chez l'être humain ou chez l'animal.

Un autre objectif essentiel de l'invention est de fournir un nouveau médicament antifongique à large spectre.

Un autre objectif essentiel de l'invention est de proposer un nouveau médicament antifongique telle que défini dans les objectifs ci-dessus et utile dans le traitement préventif et curatif des maladies fongiques, notamment les infections à *Candida albicans* et *Aspergillus fumigatus*.

Tous ces objectifs, parmi d'autres, ont été atteints par les inventeurs qui ont eu le mérite de trouver que les dérivés de $N_2$-phénylamidine présentaient de manière surprenante et inattendue une efficacité antifongique très importante et pérenne contre un large spectre d'agents infectieux à l'encontre de l'être humain ou de l'animal.

La présente invention qui satisfait en totalité ou en partie aux susdits objectifs, concerne donc en premier lieu un médicament antifongique caractérisé en ce qu'il comprend au moins un composé de formule (I) :

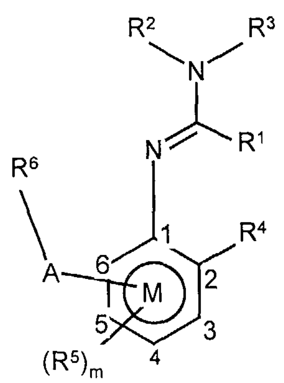

(I)

dans laquelle :

- $R^1$ est un alkyle, un alcényle, un alcynyle, un groupement monovalent carbocyclique ou hétérocyclique, chacun de ces groupements pouvant être substitué, ou l'hydrogène;
- $R^2$ et $R^3$, qui peuvent être identiques ou différents, sont l'un quelconque des groupements définiss pour $R^1$; un cyano; un acyle; $-OR^a$ ou $-SR^a$, avec $R^a$ correspondant à un alkyle, un alcényle, un alcynyle, un groupement monovalent carbocyclique ou hétérocyclique, chacun de ces groupements pouvant être substitué, ou $R^2$ et $R^3$, ou $R^2$ et $R^1$ peuvent former ensemble et avec les atomes qui les relient, un cycle pouvant être substitué;
- $R^4$ est un alkyle, un alcényle, un alcynyle, un groupement monovalent carbocyclique ou hétérocyclique, chacun de ces groupements pouvant être substitué, un groupement hydroxyle; mercapto; azido; nitro; halogèno; cyano; acyle substitué ou non substitué, amino; cyanato; thiocyanato; $-SF_5$; $-OR^a$; $-SR^a$ ou $-Si(R^a)_3$;
  - m = 0, 1, 2 ou 3;
  - l'éventuel $R^5$ ou les éventuels $R^5$, qui peuvent être identiques ou différents entre eux, répondent à la même

définition que celle donnée ci-dessus pour $R^4$;

- $R^6$ est un groupement carbocyclique ou hétérocyclique substitué ou non substitué; et
- A est une liaison directe, -O-, $-S(O)_n-$, $-NR^9-$, $-CR^7=CR^7-$, $-C\equiv C-$, $-A^1-$, $-A^1-A^1-$,$-O-(A^1)_k-O-$, $-O-(A^1)_k-$, $-A^3-$, $-A^4-$, $-A^1O-$, $-A^1S(O)_n-$, $-A^2-$, $OA^2-$,
  $-NR^9A^2-$, $-OA^2-A^1-$, $-OA^2-C(R^7)=C(R^8)-$, $-S(O)_nA^1-$, $-A^1-A^4-$,
  $- A^1-A^4-C(R^8)=N-N=CR^8-$, $-A^1-A^4-C(R^8)=N-X^2-X^3-$, $-A^1-A^4-A^3-$,
  $- A^1-A^4-N(R^9)-$, $-A^1-A^4-X-CH_2-$, $-A^1-A^4-A^1-$, $-A^1-A^4-CH_2X-$,
  $-A^1-A^4-C(R^8)=N-X^2-X^3-X^1-$, $-A^1-X-C(R^8)=N-$,
  $-A^1-X-C(R^8)=N-N=CR^8-$,$-A^1-X-C(R^8)=N-N(R^9)-$, $-A^1-X-A^--X^1-$,
  $-A^1-O-A^3-$, $-A^1-O-C(R^7)=C(R^8)-$,$-A^1-O-N(R^9)-A^2-N(R^9)-$,
  $-A^1-O-N(R^9)-A^2-$, $-A^1-N(R^9)-A^2-N(R^9)-$,$-A^1-N(R^9)-A^2-$,
  $-A^1-N(R^9)-N=C(R^8)-$, $-A^3-A^1-$, $-A^4-A^3-$, $-A^2-NR^9-$,
  $- A^1-A^2-X^1-$, $-A^1-A^1-A^2-X^1-$, $-O-A^2-N(R^9)-A^2-$, $-CR^7=CR^7-A^2-X^1-$,
  $- C\equiv C-A^2-X^1-$, $-N=C(R^8)-A^2-X^1-$, $-C(R^8)=N-N=C(R^8)-$,
  $-C(R^8)=N-N(R^9)-$, $-(CH_2)_2-O-N=C(R^8)-$ ou $-X-A^2-N(R^9)-$
  avec
  n = 0, 1 ou 2,
  k = 1 à 9,
  $A^1 = -CHR^7-$,
  $A^2 = -C(=X)-$,
  $A^3 = -C(R^8)=N-O-$,
  $A^4 = -O-N=C(R^8)-$,
  X=OouS,
  $X^1$ = O, S, $NR^9$ ou une liaison directe,
  $X^2$ = O, $NR^9$ ou une liaison directe,
  $X^3$ = hydrogène, -C(=O)-, $-SO_2-$ ou une liaison directe,

$R^7$, identiques ou différents entre eux, correspondent chacun à un alkyle substitué ou non substitué, à un cycloalkyle ou un phényle, chacun de ces groupements pouvant être substitué, l'hydrogène, un halogène, un cyano, ou un acyle;

$R^8$, identiques ou différents entre eux, correspondent chacun à un alkyle, un alcényle, un alcynyle, un alcoxy, un alkylthio, chacun de ces groupements pouvant être substitué, un groupement monovalent carbocyclique ou hétérocyclique qui peut être substitué ou non substitué, ou l'hydrogène;

$R^9$, identiques ou différents entre eux, correspondent chacun à un alkyle substitué ou non substitué, à un groupement monovalent carbocyclique ou hétérocyclique qui peut être substitué ou non substitué, ou à un acyle; ou deux groupements $R^9$ peuvent former ensemble et avec les atomes qui les relient, un cycle à 5-7 chaînons;

le groupement représenté sur le côté droit de la liaison A est relié à $R^6$;

ou $-A-R^6$ et $R^5$ forment ensemble avec le cycle benzène M, un système de cycles condensés substitués ou non substitués;

- ■ ainsi que les éventuels isomères optiques et/ou géométriques, tautomères et sels, notamment d'addition à un acide ou une base, acceptables dans le domaine pharmaceutique, de ces dérivés de formule (**I**);
- ■ et leurs mélanges.

Dans les définitions des composés de formule **(I)** exposés ci-dessus, les différents radicaux et termes chimiques employés ont, sauf précision contraire, les significations suivantes :

- "alkyle" ou alkyl-" désigne un radical hydrocarboné saturé, linéaire ou ramifié, contenant de 1 à 8 atomes de carbone ;
- "alcényle" désigne un radical hydrocarboné, linéaire ou ramifié, contenant de 1 à 8 atomes de carbone et au moins une insaturation sous forme de double liaison ;
- "alcynyle" désigne un radical hydrocarboné, linéaire ou ramifié, contenant de 1 à 8 atomes de carbone et au moins une insaturation sous forme de triple liaison ;
- "alcoxy" désigne un radical alkyl-oxy ;
- "acyle" désigne le radical formyle ou un radical alcoxycarbonyle ;
- "cycloalkyle" désigne un radical hydrocarboné cyclique saturé, contenant de 3 à 8 atomes de carbone ;
- "haloalkyle" ou haloalkyl-" désigne un radical hydrocarboné saturé, linéaire ou ramifié, contenant de 1 à 8 atomes de carbone et substitué par un plusieurs atomes d'halogène, en particulier le fluor, le chlore et le brome ;

- "aryle" désigne un radical ou plusieurs radicaux aromatiques, de préférence, un phényle ou un naphtyle ;
- "hétérocycle" désigne un radical cyclique, insaturé ou totalement ou partiellement saturé, contenant de 3 à 8 atomes, choisis parmi carbone, azote, soufre et oxygène, par exemple et de manière non limitative, pyridyle, pyridinyle, quinolyle, furyle, thiényle, pyrrolyle, oxazolinyle ;
- le terme "substitué ou non substitué" signifie que les radicaux ainsi qualifiés peuvent être substitués par un ou plusieurs radicaux choisis parmi chlore, brome, fluor, iode, alkyle, alcoxy, hydroxy, nitro, amino; cyano et acyle.

Selon un mode préféré de réalisation de l'invention, les produits **(I)** correspondent à la formule **(I)** dans laquelle :

- $R^1$ est un alkyle, un alcényle ou un alcynyle, chacun de ces groupements pouvant être substitué par un alcoxy, un haloalcoxy, un alkylthiol, un halogène ou un phényle substitué ou non substitué par un alkyle, par un haloalkyle, par un alcoxy, par un haloalcoxy, par un alkylthiol par un halogène, ou l'hydrogène;
- $R^2$ et $R^3$ qui peuvent être identiques ou différents et qui répondent à la même définition que celle donnée ci-dessus pour $R^1$ ou qui correspondent à un alcoxy, un alcoxyalkyle, un benzyloxy, un cyano ou un alkylcarbonyle;
- $R^4$ est un alkyle, un alcényle ou un alcynyle, chacun de ces groupements pouvant être substitué par un alcoxy, un haloalcoxy, un alkylthiol, un halogène ou un phényle substitué ou non substitué par un alkyle, par un haloalkyle, par un alcoxy, par un haloalcoxy, par un alkylthiol ou par un halogène; un hydroxyle; un halogène; un cyano; un acyle (de préférence : $-C(=O)R^c$, $-C(=S)R^c$ ou $-S(O)_pR^c$, avec $R^c$ correspondant à un alkyle, un haloalkyle, alcoxy, haloalcoxy, alkylthiol, une amine, une monoalkylamine, une dialkylamine ou un phényle substitué ou non substitué par un alkyle, par un haloalkyle, par un alcoxy, par un haloalcoxy, ou par un alkylthiol;
- m = 0 ou 1;
- quand il est présent, $R^5$ est un groupement répondant à la même définition que celle donnée ci-dessus pour $R^4$,
- A est une liaison directe, -O-, -S-, $-NR^9-$, $-CHR^7-$ ou $-O-CHR^7-$,

  avec $R^9$, quand il est présent correspondant à un alkyle, un alcényle ou un alcynyle, chacun de ces groupements pouvant être substitué par un alcoxy, un haloalcoxy, un alkylthiol, un halogène ou un phényle substitué ou non substitué par un alkyle, par un haloalkyle, par un alcoxy, par un haloalcoxy, par un alkylthiol ou par un halogène, ou correspond à l'hydrogène;

  et $R^7$ répond à la même definition que celle donnée ci-dessus pour $R^9$ ou représente un hydroxyle; un halogène; un cyano; un acyle; alcoxy; un haloalcoxy ou un alkylthiol;
- A est relié à la position 4 du cycle benzène M; et
- $R^6$ est un phényle ou un hétérocycle aromatique, substitué ou non substitué par un ou plusieurs substituants, identiques ou différents entre eux, et qui peuvent être sélectionnés dans la liste suivante: hydroxyle; halogène; cyano; acyle (de préférence $-C(=O)R^c$, $-C(=S)R^c$ ou $-S(O)_pR^c$, avec $R^c$ = alkyle, haloalkyle, alcoxy, haloalcoxy, alkylthiol ou phényle substitué ou non substitué par un alkyle, haloalkyle, alcoxy, haloalcoxy ou alkylthiol); amine; alkylamine; dialkylamine; alkyle, haloalkyle, $R^aO$-alkyle, acyloxyalkyle, cyanooxyalkyle, alcoxy; haloalcoxy; alkylthiol; cycloalkyle (de préférence cyclohexyle ou cyclopentyle) substitué ou non substitué par un alkyle, un haloalkyle, un alcoxy, un haloalcoxy ou par un alkylthiol; et benzyle substitué ou non substitué par un alkyle, un haloalkyle, un alcoxy, un haloalcoxy ou par un alkylthiol.

Les composés de formule **(I)** encore plus spécialement préférés sont ceux possédant les caractéristiques suivantes, prises isolément ou en combinaison :

$R^1$ = H
$R^2$ = alkyle en $C_1$-$C_6$, de préférence éthyle;
$R^3$= alkyle en $C_1$-$C_6$, de préférence méthyle;
$R^4$ = alkyle en $C_1$-$C_6$, de préférence méthyle;
$R^5$ = alkyle en $C_1$-$C_6$, de préférence méthyle et $R^5$ est relié au carbone en $C_5$ du cycle benzyle M, avec m=1;
A est relié au carbone en $C_4$ du cycle benzyle M et représente -O- ;
$R^6$ = aryle, de préférence benzyle, avantageusement substitué par au moins un alkyle et/ou par au moins un halogène ou au moins un groupement cyano.

A titre d'exemple, les composés **(I)** mis en oeuvre sont, entre autres :

- le *N*-éthyl-*N*-méthyl-*N'*-[4-(4-chloro-3-trifluorométhylphénoxy)-2,5-diméthyl phényl]-imidoformamide,
- et/ou le *N*-éthyl-*N*-méthyl-*N'*-[4-(4-fluoro-3-trifluorométhylphénoxy)-2,5-diméthylphényl]- imidoformamide,
- et/ou le *N*-éthyl-*N*-méthyl-*N'*-[4-(4-cyano-3-trifluorométhylphénoxy)-2,5-diméthylphényl]- imidoformamide,
- ainsi que les éventuels tautomères et sels, notamment d'addition à un acide ou une base, acceptables dans le

domaine pharmaceutique, de ces composés (**I**).

Selon un mode avantageux de réalisation de l'invention, le médicament antifongique comprend au moins un autre composé antifongique **(II).**

Un tel composé antifongique fait partie des composés connus de l'homme du métier et avantageusement choisi dans les familles de composés suivantes :

- les azoles, tels que le bifonazole, le butoconazole, le clotrimazole, l'eberconazole, l'econazole, le fenticonazole, le fluconazole, l'itraconazole, le kétoconazole, le miconazole, l'oxiconazole, le posaconazole, le sulconazole, le terconazole, le tioconazole, le voriconazole, le zinoconazole;
- les polyènes, tels que l'amphotéricine B, la nystatine ;
- les allylamines et les benzylamines, telles que la buténafine, la naftifine, la terbinafine ;
- les thiocarbamates, tel que tolnaftate ;
- les candines, tel que la caspofungine, la cilofungine ;
- les analogues de nucléosides, tel que la flucytosine ;
- les sordarines ;
- les polyoxines et les nikkomycines, telles que la nikkomycines Z, J, pseudo J, PX, RZ, pseudo Z ;
- les pradimicines, telles que la pradimicine A ;
- les bénanomycines ;
- les auréobasidines ;
- les UK-2A ou les UK-3A ;
- les peptides cationiques ;

pris seul ou en mélange, ainsi que leurs éventuels tautomères et sels, notamment d'addition à un acide ou une base, leurs formulations lipidiques ou liposomales, acceptables dans le domaine pharmaceutique.

Sur le plan prépondéral, il convient de préciser que conformément à l'invention le rapport massique (**I/II**) se définit comme suit:

$$0,02 \leq I/II \leq 50$$

de préférence

$$0,1 \leq I/II \leq 20$$

et plus préférentiellement encore

$$0,5 \leq I/II \leq 10.$$

Dans le cas où le composé **(II)** correspond au fluconazole ou à l'itraconazole (ou à un de leurs équivalents), il a pu être trouvé que le rapport massique (I/II) est avantageusement compris entre 0,5 et 10.

Le rapport composé **(I)**/composé **(II)** est défini comme étant le rapport en poids de ces 2 composés. Il en est de même pour tout rapport de 2 composés chimiques, ultérieurement mentionné dans le présent texte, dans la mesure où une définition différente de ce rapport n'est pas expressément indiquée.

Dans les compositions selon l'invention, le rapport composé **(I)**/composé **(II)** est avantageusement choisi de manière à produire un effet synergique. Le terme effet synergique, tel qu'on l'entend dans le présente texte est défini dans les exemples au point 2.4.

Comme cela ressort de ce qui précède, les exemples préférés d'associations synergiques selon l'invention comprendront le composé **(I),** le fluconazole et/ou l'itraconazole, ainsi que leurs éventuels tautomères et sels d'addition a un acide ou une base, pour autant que ces équivalents soient acceptables dans le domaine pharmaceutique humain ou vétérinaire.

Les domaines de rapport composé **(I)**/composé **(II)** indiqués ci-dessus ne sont nullement limitatifs de la portée de l'invention, mais sont plutôt cités à titre indicatif, l'homme du métier étant tout à fait en mesure d'effectuer des essais complémentaires pour trouver d'autres valeurs du rapport de doses de ces deux composés, pour lesquels un effet synergique est observé.

Selon une disposition préférée de l'invention la quantité d'actifs (**I/II**) présents dans les compositions fongicides

selon l'invention est comprise entre 0,5 et 99 % en poids.

Naturellement, les médicaments antifongiques selon l'invention à base d'au moins un composé **(I)** et d'au moins un composé **(II)** peuvent également comprendre un ou plusieurs autres produits actifs.

Outre ces actifs complémentaires, les médicaments antifongiques selon l'invention peuvent comporter également tout autre excipient et/ou auxiliaire utile dans les formulations pharmaceutiques.

S'agissant des présentations des médicaments selon l'invention, il y a lieu d'indiquer qu'ils se prêtent à toutes les formes galéniques connues et adaptées dans le traitement antifongique. Ainsi, ces médicaments peuvent se présenter sous forme de formulations pour l'administration par voie orale, topique, intraveineuse, intrapéritonéale.

S'agissant de la préparation des composés **(I)** on se référera à la demande de brevet internationale WO-00/46184.

Concernant la préparation des composés synergiques connus **(II),** ceux-ci sont préparés selon les règles habituelles de la pharmacopée.

Selon un autre de ces objets, l'invention se rapporte à un procédé de lutte, à titre curatif ou préventif, contre les champignons pathogènes humains ou animaux, caractérisé en ce qu'il consiste à utiliser un médicament antifongique tel que défini ci-dessus.

Les médicaments antifongiques selon l'invention contiennent habituellement de 0,5 à 99 % de la combinaison du composé **(I)** et du composé **(II)**.

La dose optimale dépend bien évidemment du type de champignon pathogène à traiter et de la gravité de l'infection.

Les champignons pathogènes cibles du médicament antifongique sont notamment ceux pris dans l'ensemble comprenant :

- le groupe des Deuteromycètes et notamment *Candida albicans, Candida tropicalis, Sporothrix schenckii, Coccidioides immitis* ;
- le groupe des Ascomycètes et notamment *Alternaria* spp, *Aspergillus fumigatus, Aspergillus flavus, Aspergillus niger, Aspergillus terreus, Cladocephalosporium* spp, *Cladosporium* spp, *Epidermophyton floccosum, Exophiala dermatitis, Fonsecaea compacta, Fonsecaea pedroso, Fusarium* spp, *Histoplasma capsulatum capsulatum, Histoplasma capsulatum buboisi, Microsporum spp, Paecilomyces* spp, *Paracoccidioides brasiliensis, Scedosporium apiospermum, Scedosporium prolificans, Scopulariopsis* spp, *Trichophyton rubrum ;*
- le groupe des Basidiomycètes et notamment *Cryptococcus neoformans neoformans, Cryptococcus neoformans gati*.

Encore un autre objet de l'invention concerne l'utilisation d'au moins un composé de formule **(I)** tel que défini *supra,* pris seul ou combinaison avec un autre composé antifongique **(II)**, pour la fabrication d'un médicament antifongique.

Avantageusement, le composé antifongique **(II)** est choisi dans les familles de composés antifongiques définis *supra.*

Encore un autre objet de l'invention concerne l'utilisation d'un médicament tel que défini ci-dessus, pour le traitement des infections d'origine fongique et notamment celles à *Candida albicans* ou *Aspergillus fumigatus*.

Les exemples suivants sont donnés à titre purement illustratifs de l'invention et ne la limitent en aucune façon.

## EXEMPLES

## MESURES IN VITRO DE L'ACTIVITE ANTIFONGIQUE DE DIFFERENTS COMPOSES UTILISES SEULS OU EN ASSOCIATION CONTRE *CANDIDA ALBICANS* ET *ASPERGILLUS FUMIGATUS*

### 1 - OBJECTIF DES ESSAIS

Les essais ont pour objectif de tester l'efficacité d'un composé de type arylamidine, ainsi que deux composés antifongiques de la famille des azoles, le fluconazole et l'itraconazole, déjà commercialisés. Ces essais visent dans un premier temps à comparer l'activité antifongique du composé de type arylamidine, pris seul, à celle des azoles. Ils ont également pour but de mettre en évidence les propriétés synergiques des associations de tels composés.

## 2 - MATERIEL ET METHODES

### 2.1 - Souches et milieux :

Les champignons suivants ont été utilisés pour cette étude : *Candida albicans* souches IP 48.72 (ATCC 10231) et *Aspergillus fumigatus* souche IP 864.64 provenant de la Collection Nationale de Cultures de Microorganismes (CNCM) de l'Institut Pasteur. Les souches sont cultivées sur un milieu agar Yeast Extract-Peptone-Dextrose (YEPD) comprenant 0,5% d'extrait de levure, 0,5% de bacto-peptone, 2% de glucose et 2% d'agar à 30°C et à l'obscurité.

### 2.2 - Les produits testés sont :

COMPOSE (1.1) : *N*-éthyl-*N*-méthyl-*N*'-[4-(4-chloro-3-trifluorométhylphénoxy)-2,5-diméthyl phényl]-imidoformamide.

→ COMPOSE (1.2) : *N*-éthyl-*N*-méthyl-*N*'-[4-(4-cyano-3-trifluorométhylphénoxy)-2,5-diméthylphényl]- imidoformamide.
→ COMPOSE (11.1) : fluconazole.
→ COMPOSE (11.2) : itraconazole.

Tous ces composés ont été préparés dans une solution de DMSO à une concentration finale de 100mg/ml. Les solutions stocks sont conservées à -20°C jusqu'à utilisation.

### 2.3 - Milieu d'essai :

Les essais sont réalisés dans un milieu RPMI 1640 sans bicarbonate de sodium, mais avec de la L-glutamine tamponnée avec 0,165 mole par litre d'acide 3-[N-Morpholino]-propanesulfonique (MOPS), enrichi (« riche ») ou non (« minimal ») avec 2% de glucose. Le pH de ce milieu est ajusté à 7,0. Le milieu est stérilisé par filtration (0,22µm) et conservé à 4°C jusqu'à utilisation.

### 2.4 - Mesure sur plaques de microtitration :

Tous les tests antifongiques sont réalisés sur plaques de microtitration. Les suspensions initiales de spores sont préparées dans une solution stérile à 0,85% de NaCl, supplémenté en Tween 80 à 0,01%. Ces suspensions initiales sont ensuite diluées dans le milieu de culture (RPMI 1640 enrichi ou non avec 2% de glucose) à une concentration finale de $10^4$ spores par ml. Les mesures de viabilité de chaque inoculum sont vérifiées par des sous-cultures d'un volume de 300 µl sur milieu agar YEPD.

Les composés antifongiques sont testés dans une gamme de concentrations allant de 0,026 à 100 µg de matière active/ml. Ces composés antifongiques sont ensuite dilués dans le milieu RPMI 1640 enrichi ou non à 2% de glucose. Une concentration finale de DMSO de 0,2% est utilisée tout au long des mesures. Chaque essai est effectué sur une série de dilutions de composés antifongiques, en doublet. Les dilutions d'antifongiques (0,1 ml) et l'inoculum fongique (0,1 ml) sont ajoutés dans chacun des puits de la plaque de microtitration. Les plaques sont lues avec un spectrophotomètre (ELX 800UV Bio-Tek Instruments, Inc) à une longueur d'ondes de 590 nm. Les plaques sont lues immédiatement après inoculation (t = 0) et après 48 heures d'incubation à 30°C et à l'obscurité. Les valeurs de densité optique obtenues sont corrélées à la croissance fongique entre les temps t = 0 et t = 48 heures. Les valeurs de densité optique sont utilisées pour calculer le pourcentage d'inhibition de la croissance pour chaque concentration d'antifongiques par comparaison avec le contrôle. Les valeurs sont alors utilisées pour tracer une courbe dose-réponse et la valeur de l'$EC_{50}$ est déterminée pour chaque champignon et chaque composé à l'aide du logiciel Grafit 5.0® (Erithacus software Ltd).

La méthode qui a été utilisée pour mesurer le type d'interaction existant entre les composés antifongiques en mélanges est la méthode de Wadley.

Dans l'approche de Wadley, la courbe dose-réponse de chacun des composés A et B, et du mélange AB est construite. L'$EC_{50}$ est calculée pour chaque composé pris individuellement et pour le mélange. Si a et b sont les quantités absolues des composés A et B dans le mélange (a=1, b=1, a + b = 2 dans nos conditions), la concentration efficace attendue ($EC_{50exp}$) peut être calculée de la manière suivante :

$$EC_{50exp} = (a + b)/[a/EC_{50A}) + b/(EC_{50B})$$

L'approche de Wadley peut être utilisée pour estimer le type d'interaction existant entre les composés fongicides, quelle que soit leur concentration. Sa fiabilité n'est pas dépendante du pourcentage d'inhibition. Le type d'interaction entre deux composés est donné par le niveau de l'interaction (NI) qui correspond au ratio entre la concentration efficace attendue ($EC_{50exp}$) et celle observée ($EC_{50obs}$) du mélange. La nature de l'interaction obtenue par la formule de Wadley est présentée dans le tableau 1 (voir U. Gisi, Synergistic interaction in fungicide mixtures, 1996. Phytopathology 86, 1273-1279) ci-dessous.

| Niveau d'interaction | Définition mathématique | Définition biologique |
|---|---|---|
| <1 | Antagoniste | |
| 1 | Additive | |
| >1 | Synergique | |
| <0,5 | | Antagoniste |
| 0,5 - 1 | | Additive |
| >1,5 | | Synergique |

**3-RESULTATS**

Tableau 1 :

| Efficacité *in vitro* du composé 1.1, du composé 11.1 et du composé 11.2 utilisés seuls contre *Aspergillus fumigatus* cultivé sur milieu minimal RPMI 1640 (MM). | | | | | | | |
|---|---|---|---|---|---|---|---|
| % inhibition$^{\alpha}$ | Dose (µg/ml) | | | | | | |
| | 0,026 | 0,098 | 0,39 | 1,562 | 6,25 | 25 | 100 |
| Composé I.1 | 1 | 57,9 | 80,1 | 94,6 | 97,1 | 98,4 | 98,1 |
| Composé II.1 | 4,8 | 4,8 | 5,8 | 8,7 | 7,7 | 23,1 | 41,4 |
| Composé II.2 | 2,2 | 31,8 | 49,3 | 99,6 | 100 | 100 | 99 |

α Le pourcentage d'inhibition de la croissance est déterminé après 48 heures d'incubation à 30°C et à l'obscurité.

Tableau 2 :

| Efficacité *in vitro* du composé I.1, du composé 11.1 et du composé 11.2 utilisés seuls contre *Aspergillus fumigatus* cultivé sur milieu riche RPMI 1640 (MR). | | | | | | | |
|---|---|---|---|---|---|---|---|
| % inhibition$^{\alpha}$ | Dose (µg/ml) | | | | | | |
| | 0,026 | 0,098 | 0,39 | 1,562 | 6,25 | 25 | 100 |
| Composé I.1 | 2,24 | 23,1 | 51,1 | 97,4 | 98,1 | 98,1 | 97,4 |
| Composé II.1 | 11,2 | 20,7 | 25,9 | 27,6 | 28,4 | 48,3 | 46,6 |
| Composé II.2 | 18,6 | 47,5 | 88,6 | 99,2 | 100 | 100 | 100 |

α Le pourcentage d'inhibition de la croissance est déterminé après 48 heures d'incubation à 30°C et à l'obscurité.

Tableau 3 :

| Efficacité *in vitro* du composé I.1, du composé II.1 et du composé 11.2 utilisés seuls contre *Candida albicans* cultivé sur milieu minimal RPMI 1640 (MM). | | | | | | | |
|---|---|---|---|---|---|---|---|
| % inhibition$^{\alpha}$ | Dose (µg/ml) | | | | | | |
| | 0,026 | 0,098 | 0,39 | 1,562 | 6,25 | 25 | 100 |
| Composé I.1 | 4,6 | 7 | 46,1 | 96,2 | 98,7 | 99,2 | 98,1 |
| Composé II.1 | 2,1 | 9,7 | 38,5 | 64,7 | 77,7 | 81,9 | 91,6 |
| Composé II.2 | 58,1 | 75,4 | 78,2 | 74,6 | 78,6 | 87,9 | 95,2 |

α Le pourcentage d'inhibition de la croissance est déterminé après 48 heures d'incubation à 30°C et à l'obscurité.

Tableau 4:

| Efficacité *in vitro* du composé 1.1, du composé II.1 et du composé 11.2 utilisés seuls contre *Candida albicans* cultivé sur milieu riche RPMI 1640 (MR). | | | | | | | |
|---|---|---|---|---|---|---|---|
| % inhibition$^{\alpha}$ | Dose (µg/ml) | | | | | | |
| | 0,026 | 0,098 | 0,39 | 1,562 | 6,25 | 25 | 100 |
| Composé I.1 | 12,6 | 27,3 | 43,6 | 96,9 | 99,5 | 99,5 | 98,8 |
| Composé II.1 | 11,1 | 32,5 | 72,6 | 86,8 | 94 | 95,3 | 36,1 |
| Composé II.2 | 44,7 | 85,1 | 86,2 | 85,1 | 86,2 | 95,2 | 95,2 |

α Le pourcentage d'inhibition de la croissance est déterminé après 48 heures d'incubation à 30°C et à l'obscurité.

Tableau 5:

| Efficacité$^{\alpha}$ *in vitro* du composé I.1, du Composé 11.1 et du composé 11.2 utilisés seuls contre *Aspergillus fumigatus* et *Candida albicans* cultivés sur un milieu RPMI 1640 minimal (MM) et sur un milieu riche RPMI 1640 (MR). | | | | |
|---|---|---|---|---|
| Fongicide | $EC_{50}$ (µg/ml) | | | |
| | *Aspergillus fumigatus* | | *Candida albicans* | |
| | MM | MR | MM | MR |
| Composé I.1 | 0,1 | 0,31 | 0,41 | 0,3 |
| Composé II.1 | 575$^{\beta}$ | 125$^{\beta}$ | 0,96 | 0,19 |
| Composé II.2 | 0,27 | 0,27 | 0,022 | 0,017 |

α Le pourcentage d'inhibition de la croissance est déterminé après 48 heures d'incubation à 30 ºC à l'obscurité.

β Ces $EC_{50}$ sont extrapolées à partir de l'analyse des courbes dose-réponse obtenues à l'aide du logiciel Grafit 5.0®.

Tableau 6:

| Evaluation *in vitro* de l'importance de l'interaction entre le composé 1.1, le composé II.1 et le composé 11.2 en utilisant la formule de Wadley contre *Aspergillus fumigatus* et *Candida albicans* cultivés sur milieu minimal (MM) et milieu riche RPMI 1640 (MR). | | | | |
|---|---|---|---|---|
| Fongicide | *Aspergillus fumigatus* | | *Candida albicans* | |
| | MM | MR | MM | MR |
| Mélange I.1 + II.1 | | | | |
| $EC_{50\ obs}$<br>$EC_{50\ exp}$<br>N.I.[α] | 0,200<br>0,21<br>1,06 | 0.122<br>0,62<br>**5,09** | 0,225<br>0,573<br>**2,54** | 0,451<br>0,223<br>0.494 |
| Mélange I.1 + II.2 | | | | |
| $EC_{50obs}$<br>$EC_{50exp}$<br>N.I. | 0,270<br>0,15<br>0,56 | 0,175<br>0,292<br>**1,67** | 0,112<br>0,042<br>0,376 | 0,129<br>0,032<br>0,25 |

α Le niveau d'interaction (N.I.) correspond au ratio de la concentration efficace attendue ($EC_{50exp}$) sur la concentration efficace observée ($EC_{50obs}$) du mélange. L'interaction synergique est présente lorsque le niveau d'interaction est supérieur à 1,5 (valeurs en **gras**).

**4 - CONCLUSIONS**

Les différents résultats obtenus et présentés ci-dessus démontrent l'efficacité du composé I.1, que ce soit sur milieu RPMI 1640 minimal (MM) ou sur milieu riche (MR), contre *Aspergillus fumigatus* et *Candida albicans* avec des $EC_{50}$ comprise entre 0,1 et 0,5 µg/ml, et donc ayant une activité équivalente à l'itraconazole (composé II.2) contre *Aspergillus fumigatus* et une activité au moins équivalente au fluconazole (composé II.1) contre *Candida albicans*.

En ce qui concerne les interactions entre composés, les résultats obtenus par la méthode de Wadley montrent que l'association du composé I.1 et du fluconazole (composé II.1) présente des effets synergiques surprenants tant vis à vis *d'Aspergillus fumigatus* que de *Candida albicans*. Le médicament antifongique selon l'invention constitue donc une réelle avancée en terme d'amélioration de l'activité antifongique par rapport aux références du marché.

Lors d'une seconde étude le composé I.2 selon l'invention a été testé. Il s'agit du : *N*-éthyl-*N*-méthyl-*N'*-[4-(4-cyano-3-trifluorométhylphénoxy)-2,5-diméthylphényl]- imidoformamide

Le composé (I.1) selon l'invention ainsi que le fluconazole (II.1) et l'itraconazole (II.2) ont également été *testés in vitro* sur *Candida albicans* et *Aspergillus fumigatus* en milieu enrichi.

Les conditions expérimentales sont les mêmes que précédemment décrites.

**Résultats**

Tableau 1.

| Efficacité[α] *in vitro* des composés I.1, I.2, II.1 et II.2, utilisés seuls contre *Candida albicans* et *Aspergillus fumigatus* en milieu RPMI 1640 enrichi à 2% de glucose. | | |
|---|---|---|
| Fungicide | $EC_{50}$[χ] (µg/ml) | |
| | *Candida albicans* | *Aspergillus fumigatus* |
| | IP 48.72[β] | IP 864.64[β] |
| Compound I.1 | 0,635 | 0,179 |
| Compound I.2 | 0,205 | 0,088 |
| Compound II.1 | 0,354 | 125 |
| Compound II.2 | 0,082 | 0,187 |

α Le pourcentage d'inhibition de la croissance est déterminé après 48 heures d'incubation à 30°C et dans l'obscurité.
β Les souches IP48.72 et IP864.64 ont servi de références pour ces expériences.
χ Ces $EC_{50}$ sont déterminées après analyses des courbes dose-réponse obtenues au moyen du logiciel Grafit 5.0®.

Sur *Candida albicans*, l'$EC_{50}$ du composé I.1 est 1,8 et 7,7 fois supérieure à celle des composés II.1 et II.2, tandis que le composé I.2 montre une meilleure efficacité *in vitro* contre *Candida albicans* que les composés I.1 et II.1.

Sur *Aspergillus fumigatus* (IP 864.64), l'$EC_{50}$ du composé I.1 est environ 700 fois inférieure à celle du composé II.1 et similaire à celle du composé II.2, tandis que le composé I.2 montre une efficacité *in vitro* sur *Aspergillus fumigatus* 2 fois supérieure à celle des composés I.1 et II.2.

## Revendications

1. Médicament antifongique **caractérisé en ce qu'**il comprend au moins un composé de formule (I) :

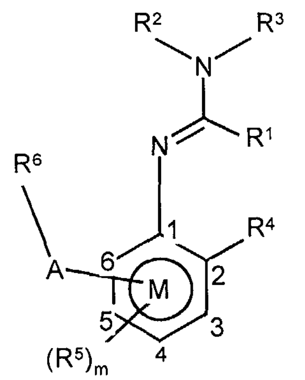

(I)

dans laquelle :

- $R^1$ est un alkyle, un alcényle, un alcynyle, un groupement monovalent carbocyclique ou hétérocyclique, chacun de ces groupements pouvant être substitué, ou l'hydrogène;
- $R^2$ et $R^3$, qui peuvent être identiques ou différents, sont l'un quelconque des groupements définis pour $R^1$; un cyano; un acyle; $-OR^a$ ou $-SR^a$, avec $R^a$ correspondant à un alkyle, un alcényle, un alcynyle, un groupement monovalent carbocyclique ou hétérocyclique, chacun de ces groupements pouvant être substitué, ou $R^2$ et $R^3$, ou $R^2$ et $R^1$ peuvent former ensemble et avec les atomes qui les relient, un cycle pouvant être substitué;
- $R^4$ est un alkyle, un alcényle, un alcynyle, un groupement monovalent carbocyclique ou hétérocyclique, chacun de ces groupements pouvant être substitué, un groupement hydroxyle; mercapto; azido; nitro; halogèno; cyano; acyle substitué ou non substitué, amino; cyanato; thiocyanato; $-SF_5$; $-OR^a$; $-SR^a$ ou $-Si(R^a)_3$;

  - m = 0, 1, 2 ou 3;
  - l'éventuel $R^5$ ou les éventuels $R^5$, qui peuvent être identiques ou différents entre eux, répondent à la même définition que celle donnée ci-dessus pour $R^4$;
  - $R^6$ est un groupement carbocyclique ou hétérocyclique substitué ou non substitué; et
  - A est une liaison directe, -O-, $-S(O)_n-$, $-NR^9-$, $-CR^7=CR^7-$, -C=C-, $-A^1-$, $-A^1-A^1-$,$-O-(A^1)_k-O-$, $-O-(A^1)_k-$, $-A^3-$, $-A^4-$, $-A^1O-$, $-A^1S(O)_n-$, $-A^2-$, $OA^2-$,

    $-NR^9A^2-$, $-OA^2-A^1-$, $-OA^2-C(R^7)=C(R^8)-$, $-S(O)_nA^1-$, $-A^1-A^4-$,
    $-A^1-A^4-C(R^8)=N-N=CR^8-$, $-A^1-A^4-C(R^8)=N-X^2-X^3-$, $-A^1-A^4-A^3-$,
    $- A^1-A^4-N(R^9)-$, $-A^1-A^4-X-CH_2-$, $-A^1-A^4-A^1-$, $-A^1-A^4-CH_2X-$,
    $-A^1-A^4-C(R^8)=N-X^2-X^3-X^1-$, $-A^1-X-C(R^8)=N-$,
    $- A^1-X-C(R^8)=N-N=CR^8-$,$-A^1-X-C(R^8)=N-N(R^9)-$, $-A^1-X-A^{-}-X^1-$,
    $-A^1-O-A^3-$, $-A^1-O-C(R^7)=C(R^8)-$,$-A^1-O-N(R^9)-A^2-N(R^9)-$,
    $-A^1-O-N(R^9)-A^2-$, $-A^1-N(R^9)-A^2-N(R^9)-$,$-A^1-N(R^9)-A^2-$,
    $-A^1-N(R^9)-N=C(R^8)-$, $-A^3-A^1-$, $-A^4-A^3-$, $-A^2-NR^9-$,
    $- A^1-A^2-X^1-$, $-A^1-A^1-A^2-X^1-$, $-O-A^2-N(R^9)-A^2-$, $-CR^7=CR^7-A^2-X^1-$,
    $- C{\equiv}C-A^2-X^1-$, $-N=C(R^8)-A^2-X^1-$, $-C(R^8)=N-N=C(R^8)-$,
    $-C(R^8)=N-N(R^9)-$, $-(CH_2)_2-O-N=C(R^8)-$ ou $-X-A^2-N(R^9)-$
    avec
    n = 0, 1 ou 2,

k = 1 à 9,
$A^1$ = -$CHR^7$-,
$A^2$ = -C(=X)-,
$A^3$ = -C($R^8$)=N-O-,
$A^4$ = -O-N=C($R^8$)-,
X = O ou S,
$X^1$ = O, S, $NR^9$ ou une liaison directe,
$X^2$ = O, $NR^9$ ou une liaison directe,
$X^3$ = hydrogène, -C(=O)-, -$SO_2$- ou une liaison directe,

$R^7$, identiques ou différents entre eux, correspondent chacun à un alkyle substitué ou non substitué, à un cycloalkyle ou un phényle, chacun de ces groupements pouvant être substitué, l'hydrogène, un halogène, un cyano, ou un acyle;

$R^8$, identiques ou différents entre eux, correspondent chacun à un alkyle, un alcényle, un alcynyle, un alcoxy, un alkylthio, chacun de ces groupements pouvant être substitué, un groupement monovalent carbocyclique ou hétérocyclique qui peut être substitué ou non substitué, ou l'hydrogène;

$R^9$, identiques ou différents entre eux, correspondent chacun à un alkyle substitué ou non substitué, à un groupement monovalent carbocyclique ou hétérocyclique qui peut être substitué ou non substitué, ou à un acyle; ou deux groupements $R^9$ peuvent former ensemble et avec les atomes qui les relient, un cycle à 5-7 chaînons;

le groupement représenté sur le côté droit de la liaison A est relié à $R^6$;

ou -A-$R^6$ et $R^5$ forment ensemble avec le cycle benzène M, un système de cycles condensés substitués ou non substitués;

- ainsi que les éventuels isomères optiques et/ou géométriques, tautomères et sels, notamment d'addition à un acide ou une base, acceptables dans le domaine pharmaceutique, de ces dérivés de formule (**I**);
- et leurs mélanges.

**2.** Médicament selon la revendication 1, **caractérisé en ce que** :

- $R^1$ est un alkyle, un alcényle ou un alcynyle, chacun de ces groupements pouvant être substitué par un alcoxy, un haloalcoxy, un alkylthiol, un halogène ou un phényle substitué ou non substitué par un alkyle, par un haloalkyle, par un alcoxy, par un haloalcoxy, par un alkylthiol par un halogène, ou l'hydrogène;
- $R^2$ et $R^3$ qui peuvent être identiques ou différents, et qui répondent à la même définition que celle donnée ci-dessus pour $R^1$ ou qui correspondent à un alcoxy, un alcoxyalkyle, un benzyloxy, un cyano ou un alkylcarbonyle;
- $R^4$ est un alkyle, un alcényle ou un alcynyle, chacun de ces groupements pouvant être substitué par un alcoxy, un haloalcoxy, un alkylthiol, un halogène ou un phényle substitué ou non substitué par un alkyle, par un haloalkyle, par un alcoxy, par un haloalcoxy, par un alkylthiol ou par un halogène; un hydroxyle; un halogène; un cyano; un acyle, une amine, une monoalkylamine, une dialkylamine ou un phényle substitué ou non substitué par un alkyle, par un haloalkyle, par un alcoxy, par un haloalcoxy, ou par un alkylthiol;
- m = 0 ou 1;
- quand il est présent, $R^5$ est un groupement répondant à la même définition que celle donnée ci-dessus pour $R^4$,
- A est une liaison directe, -O-, -S-, -$NR^9$-, -$CHR^7$- ou -O-$CHR^7$-,

  avec $R^9$, quand il est présent correspondant à un alkyle, un alcényle ou un alcynyle, chacun de ces groupements pouvant être substitué par un alcoxy, un haloalcoxy, un alkylthiol, un halogène ou un phényle substitué ou non substitué par un alkyle, par un haloalkyle, par un alcoxy, par un haloalcoxy, par un alkylthiol ou par un halogène, ou correspond à l'hydrogène ;

  et $R^7$ répond à la même définition que celle donnée ci-dessus pour $R^9$ ou représente un hydroxyle; un halogène; un cyano; un acyle; alcoxy; un haloalcoxy ou un alkylthiol;
- A est relié à la position 4 du cycle benzène M; et
- $R^6$ est un phényle ou un hétérocycle aromatique, substitué ou non substitué par un ou plusieurs substituants, identiques ou différents entre eux, et qui peuvent être sélectionnés dans la liste suivante: hydroxyle; halogène; cyano; acyle; amine; alkylamine; dialkylamine; alkyle, haloalkyle, $R^aO$-alkyle, acyloxyalkyle, cyanooxyalkyle, alcoxy; haloalcoxy; alkylthiol; cycloalkyle substitué ou non substitué par un alkyle, un haloalkyle, un alcoxy, un haloalcoxy ou par un alkylthiol; et benzyle substitué ou non substitué par un alkyle, un haloalkyle, un alcoxy, un haloalcoxy ou par un alkylthiol.

**3.** Médicament selon la revendication 1, **caractérisé en ce que** :

- $R^1$ = H
- $R^2$ = alkyle en $C_1$-$C_6$, de préférence éthyle;
- $R^3$ = alkyle en $C_1$-$C_6$, de préférence méthyle;
- $R^4$ = alkyle en $C_1$-$C_6$, de préférence méthyle;
- $R^5$ = alkyle en $C_1$-$C_6$, de préférence méthyle et $R^5$ est relié au carbone en $C_5$ du cycle benzyle M, avec m=1;
- A est relié au carbone en $C_4$ du cycle benzyle M et représente -O- ;
- $R^6$ = aryle, de préférence benzyle, avantageusement substitué par au moins un alkyle et/ou par au moins un halogène.

**4.** Médicament selon la revendication 3, **caractérisé en ce que** le composé (**I**) est :

- le *N*-éthyl-*N*-méthyl-*N*'-[4-(4-chloro-3-trifluorométhylphénoxy)-2,5-diméthyl phényl]-imidoformamide,
- et/ou le *N*-éthyl-*N*-méthyl-*N*'-[4-(4-fluoro-3-trifluorométhylphénoxy)-2,5-diméthylphényl]- imidoformamide,
- et/ou le *N*-éthyl-*N*-méthyl-*N*'-[4-(4-cyano-3-trifluorométhylphénoxy)-2,5-diméthylphényl]- imidoformamide,

ainsi que les éventuels tautomères et sels, notamment d'addition à un acide ou une base, acceptables dans le domaine pharmaceutique, de ces composés (**I**).

**5.** Médicament selon l'une des revendications 1 à 4, **caractérisé en ce qu'**il comprend en outre au moins un autre composé antifongique (**II**).

**6.** Médicament selon la revendication précédente, **caractérisé en ce que** le composé antifongique (**II**) est choisi dans les familles d'antifongiques suivantes :

- les azoles, tels que le bifonazole, le butoconazole, le clotrimazole, l'eberconazole, l'econazole, le fenticonazole, le fluconazole, l'itraconazole, le kétoconazole, le miconazole, l'oxiconazole, le posaconazole, le sulconazole, le terconazole, le tioconazole, le voriconazole, le zinoconazole;
- les polyènes, tels que l'amphotéricine B, la nystatine ;
- les allylamines et les benzylamines, telles que la buténafine, la naftifine, la terbinafine ;
- les thiocarbamates, tel que tolnaftate ;
- les candines, tel que la caspofungine, la cilofungine ;
- les analogues de nucléosides, tel que la flucytosine ;
- les sordarines ;
- les polyoxines et les nikkomycines, telles que la nikkomycines Z, J, pseudo J, PX, RZ, pseudo Z ;
- les pradimicines, telles que la pradimicine A ;
- les bénanomycines ;
- les auréobasidines ;
- les UK-2A ou les UK-3A ;
- les peptides cationiques ;

pris seul ou en mélange, ainsi que leurs éventuels tautomères et sels, notamment d'addition à un acide ou une base, leurs formulations lipidiques ou liposomales, acceptables dans le domaine pharmaceutique.

**7.** Médicament antifongique selon la revendication 4 ou 5, **caractérisé en ce que** le rapport massique **(I/II)** se définit comme suit:

$$0{,}02 \leq I/II \leq 50$$

de préférence

$$0{,}1 \leq I/II \leq 20$$

et plus préférentiellement encore

$$0{,}5 \leq I/II \leq 10$$

**8.** Médicament antifongique selon l'une des revendications 4 ou 5, **caractérisé en ce que** le rapport composé (**I**)/composé (**II**) est choisi de manière à produire un effet synergique.

**9.** Médicament antifongique selon la revendication précédente, **caractérisé en ce que** le rapport composé (**I**)/composé (**II**) est compris entre 0,5 et 10.

**10.** Médicament antifongique selon l'une des revendications précédentes, **caractérisé en ce qu'**il comprend en outre au moins un excipient pharmaceutiquement acceptable.

**11.** Médicament antifongique selon l'une des revendications précédentes, **caractérisé en ce qu'**il comprend de 0,5 à 99% de la combinaison du composé (**I**) et du composé (**II**).

**12.** Utilisation, pour la fabrication d'un médicament antifongique, d'au moins un composé de formule (**I**)

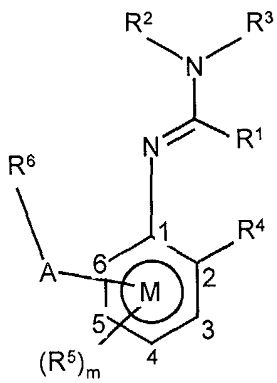

(I)

dans laquelle :

- $R^1$ est un alkyle, un alcényle, un alcynyle, un groupement monovalent carbocyclique ou hétérocyclique, chacun de ces groupements pouvant être substitué, ou l'hydrogène;
- $R^2$ et $R^3$, qui peuvent être identiques ou différents, sont l'un quelconque des groupements définis pour $R^1$; un cyano; un acyle; $-OR^a$ ou $-SR^a$, avec $R^a$ correspondant à un alkyle, un alcényle, un alcynyle, un groupement monovalent carbocyclique ou hétérocyclique, chacun de ces groupements pouvant être substitué, ou $R^2$ et $R^3$, ou $R^2$ et $R^1$ peuvent former ensemble et avec les atomes qui les relient, un cycle pouvant être substitué;
- $R^4$ est un alkyle, un alcényle, un alcynyle, un groupement monovalent carbocyclique ou hétérocyclique, chacun de ces groupements pouvant être substitué, un groupement hydroxyle; mercapto; azido; nitro; halogèno; cyano; acyle substitué ou non substitué, amino; cyanato; thiocyanato; $-SF_5$; $-OR^a$; $-SR^a$ ou $-Si(R^a)_3$;

  - m = 0, 1, 2 ou 3;
  - l'éventuel $R^5$ ou les éventuels $R^5$, qui peuvent être identiques ou différents entre eux, répondent à la même définition que celle donnée ci-dessus pour $R^4$;
  - $R^6$ est un groupement carbocyclique ou hétérocyclique substitué ou non substitué; et
  - A est une liaison directe, -O-, $-S(O)_n-$, $-NR^9-$, $-CR^7=CR^7-$, $-C\equiv C-$, $-A^1-$, $-A^1-A^1-$,$-O-(A^1)_k-O-$, $-O-(A^1)_k-$, $-A^3-$, $-A^4-$, $-A^1O-$, $-A^1S(O)_n-$, $-A^2-$, $OA^2-$,
    $- NR^9A^2-$, $-OA^2-A^1-$, $-OA^2-C(R^7)=C(R^8)-$, $-S(O)_nA^1-$, $-A^1-A^4-$,
    $- A^1-A^4-C(R^8)=N-N=CR^8-$, $-A^{1-}A^4-C(R^8)=N-X^2-X^3-$, $-A^1-A^4-A^3-$,
    $-A^1-A^4-N(R^9)-$, $-A^1-A^4-X-CH_2-$, $-A^1-A^4-A^1-$, $-A^1-A^4-CH_2X-$,
    $-A^1-A^4-C(R^8)=N-X^2-X^3-X^1-$, $-A^1-X-C(R^8)=N-$,
    $-A^1-X-C(R^8)=N-N=CR^8-$,$-A^1-X-C(R^8)=N-N(R^9)-$, $-A^1-X-A^--X^1-$,
    $-A^1-O-A^3-$,$-A^1-O-C(R^7)=C(R^8)-$,$-A^1-O-N(R^9)-A^2-N(R^9)-$,
    $-A^1-O-N(R^9)-A^2-$, $-A^1-N(R^9)-A^2-N(R^9)-$,$-A^1-N(R^9)-A^2-$,
    $-A^1-N(R^9)-N=C(R^8)-$, $-A^3-A^1-$, $-A^4-A^3-$, $-A^2-NR^9-$,
    $-A^1-A^2-X^1-$, $-A^1-A^1-A^2-X^1-$, $-O-A^2-N(R^9)-A^2-$, $-CR^7=CR^7-A^2-X^1-$,
    $-C\equiv C-A^2-X^1-$, $-N=C(R^8)-A^2-X^1-$, $-C(R^8)=N-N=C(R^8)-$,

$-C(R^8)=N-N(R^9)-$, $-(CH_2)_2-O-N=C(R^8)-$ ou $-X-A^2-N(R^9)-$
avec
n = 0, 1 ou 2,
k = 1 à 9,
$A^1 = -CHR^7-$,
$A^2 = -C(=X)-$,
$A^3 = -C(R^8)=N-O-$,
$A^4 = -O-N=C(R^8)-$,
X = O ou S,
$X^1$ = O, S, $NR^9$ ou une liaison directe,
$X^2$ = O, $NR^9$ ou une liaison directe,
$X^3$ = hydrogène, -C(=O)-, $-SO_2-$ ou une liaison directe,

$R^7$, identiques ou différents entre eux, correspondent chacun à un alkyle substitué ou non substitué, à un cycloalkyle ou un phényle, chacun de ces groupements pouvant être substitué, l'hydrogène, un halogène, un cyano, ou un acyle;

$R^8$, identiques ou différents entre eux, correspondent chacun à un alkyle, un alcényle, un alcynyle, un alcoxy, un alkylthio, chacun de ces groupements pouvant être substitué, un groupement monovalent carbocyclique ou hétérocyclique qui peut être substitué ou non substitué, ou l'hydrogène;

$R^9$, identiques ou différents entre eux, correspondent chacun à un alkyle substitué ou non substitué, à un groupement monovalent carbocyclique ou hétérocyclique qui peut être substitué ou non substitué, ou à un acyle; ou deux groupements $R^9$ peuvent former ensemble et avec les atomes qui les relient, un cycle à 5-7 chaînons;

le groupement représenté sur le côté droit de la liaison A est relié à $R^6$;

ou $-A-R^6$ et $R^5$ forment ensemble avec le cycle benzène M, un système de cycles condensés substitués ou non substitués;

- ainsi que les éventuels isomères optiques et/ou géométriques, tautomères et sels, notamment d'addition à un acide ou une base, acceptables dans le domaine pharmaceutique, de ces dérivés de formule (**I**);
- et leurs mélanges.

ledit composé (**I**) étant pris seul ou en combinaison avec un autre composé antifongique (**II**).

**13.** Utilisation selon la revendication précédente, **caractérisé en ce que** le composé antifongique **(II)** est choisi dans les familles d'antifongiques suivantes :

- les azoles, tels que le bifonazole, le butoconazole, le clotrimazole, l'eberconazole, l'econazole, le fenticonazole, le fluconazole, l'itraconazole, le kétoconazole, le miconazole, l'oxiconazole, le posaconazole, le sulconazole, le terconazole, le tioconazole, le voriconazole, le zinoconazole;
- les polyènes, tels que l'amphotéricine B, la nystatine ;
- les allylamines et les benzylamines, telles que la buténafine, la naftifine, la terbinafine ;
- les thiocarbamates, tel que tolnaftate ;
- les candines, tel que la caspofungine, la cilofungine ;
- les analogues de nucléosides, tel que la flucytosine ;
- les sordarines ;
- les polyoxines et les nikkomycines, telles que la nikkomycines Z, J, pseudo J, PX, RZ, pseudo Z ;
- les pradimicines, telles que la pradimicine A ;
- les bénanomycines ;
- les auréobasidines ;
- les UK-2A ou les UK-3A ;
- les peptides cationiques ;

pris seul ou en mélange, ainsi que leurs éventuels tautomères et sels, notamment d'addition à un acide ou une base, leurs formulations lipidiques ou liposomales, acceptables dans le domaine pharmaceutique.

**14.** Utilisation d'un médicament antifongique selon l'une des revendications 1 à 11, pour le traitement des infections à *Candida albicans*.

**15.** Utilisation d'un médicament antifongique selon l'une des revendications 1 à 11, pour le traitement des infections

à *Aspergillus fumigatus*.

Office européen des brevets

**RAPPORT PARTIEL DE RECHERCHE EUROPEENNE**
qui selon la règle 45 de la Convention sur le brevet européen est consideré, aux fins de la procédure ultérieure, comme le rapport de la recherche européenne

Numéro de la demande
EP 02 35 6210

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X,D | WO 00 46184 A (HOECHST SCHERING AGREVO GMBH ;ATHERALL JOHN FREDERICK (GB); HOUGH) 10 août 2000 (2000-08-10)<br>composé 364 (page 46) et composé 393 (page 47)<br>* revendication 1 *<br>* page 10, ligne 10 - ligne 15 *<br>--- | 1-15 | A61K31/155 |
| X | EP 1 178 038 A (AVENTIS CROPSCIENCE SA) 6 février 2002 (2002-02-06)<br>* page 5, ligne 56 *<br>* alinéa [0034]; revendications 1-33 *<br>----- | 1-3,5-15 | |

DOMAINES TECHNIQUES RECHERCHES (Int.Cl.7)
A61K

## RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

| Lieu de la recherche | Date d'achèvement de la recherche | Examinateur |
|---|---|---|
| MUNICH | 18 mars 2003 | Loher, F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C08)

Office européen des brevets

RECHERCHE INCOMPLETE
FEUILLE SUPPLEMENTAIRE C

Numéro de la demande
EP 02 35 6210

Bien que les revendications 14 et 15 concernent une méthode de traitement du corps humain/animal (Article 52(4) CEB), la recherche a été effectuée et basée sur les effets imputés au produit.

--------------------

Revendications ayant fait
l'objet de recherches incomplètes:
14,15

Raison pour la limitation de la recherche (invention(s) non brevetable(s)):

Article 52 (4) CBE - Méthode de traitement thérapeutique du corps humain ou animal

--------------------

Limitation additionnelle de la recherche

Revendications ayant fait
l'objet de recherches complètes:
4

Revendications ayant fait
l'objet de recherches incomplètes:
1-3,5-15

Raison pour la limitation de la recherche:

Les revendications 1-3 et 5-15 présentes ont trait à une très grande variété de composés. En fait, les revendications contiennent tant d'options et de variables qu'une recherche significative de l'objet des revendications devient impossible. Par conséquent, la recherche a été effectuée pour les parties de la demande qui apparaissent être concises, c'est à dire pour revendication 4 et les exemples.

**ANNEXE AU RAPPORT DE RECHERCHE EUROPEENNE RELATIF A LA DEMANDE DE BREVET EUROPEEN NO.** EP 02 35 6210

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

18-03-2003

| Document brevet cité au rapport de recherche | | Date de publication | Membre(s) de la famille de brevet(s) | | | Date de publication |
|---|---|---|---|---|---|---|
| WO 0046184 | A | 10-08-2000 | AU | 2308800 | A | 25-08-2000 |
| | | | BR | 0009314 | A | 13-02-2002 |
| | | | CA | 2360943 | A1 | 10-08-2000 |
| | | | CN | 1342137 | T | 27-03-2002 |
| | | | CZ | 20012842 | A3 | 13-03-2002 |
| | | | EP | 1150944 | A1 | 07-11-2001 |
| | | | WO | 0046184 | A1 | 10-08-2000 |
| | | | HU | 0105098 | A2 | 29-04-2002 |
| | | | JP | 2002536354 | T | 29-10-2002 |
| | | | TR | 200102237 | T2 | 21-12-2001 |
| EP 1178038 | A | 06-02-2002 | EP | 1178038 | A1 | 06-02-2002 |
| | | | EP | 1179528 | A2 | 13-02-2002 |
| | | | JP | 2002193909 | A | 10-07-2002 |
| | | | US | 2002082267 | A1 | 27-06-2002 |

EPO FORM P0460

Pour tout renseignement concernant cette annexe : voir Journal Officiel de l'Office européen des brevets, No.12/82